# NEUE EUROPÄISCHE PATENTSCHRIFT

(11) **EP 0 683 657 B2**
(45) Veröffentlichungstag und Bekanntmachung des Hinweises auf die Entscheidung über den Einspruch: **15.06.2005**
(45) Hinweis auf die Patenterteilung: 29.05.2002
(21) Anmeldenummer: 94906854.8
(22) Anmeldetag: 09.02.1994
(51) Int. Cl.: A61H 23/00, A61N 1/18

(54) **GERÄT ZUR SCHMERZTHERAPIE UND/ODER ZUR BEEINFLUSSUNG DES VEGETATIVEN NERVENSYSTEMS**
APPARATUS FOR ANALGESIC THERAPY AND/OR FOR INFLUENCING THE VEGETATIVE NERVOUS SYSTEM
APPAREIL DE THERAPIE ANALGESIQUE ET/OU PERMETTANT D'AGIR SUR LE SYSTEME NERVEUX VEGETATIF

(30) Priorität: 10.02.1993 DE 4303830; 03.12.1993 DE 4341323
(43) Veröffentlichungstag der Anmeldung: 29.11.1995
(73) Patentinhaber: SIEMENS AKTIENGESELLSCHAFT, 80333 München (DE)
(72) Erfinder: WETH, Gosbert, D-91056 Erlangen (DE); WILHELM, Gunter, D-91056 Erlangen (DE)
(86) Internationale Anmeldenummer: PCT/DE1994/000133
(87) Internationale Veröffentlichungsnummer: WO 1994/017771

(56) Entgegenhaltungen:
- EP-A- 0 301 360
- WO-A-85/03234
- US-A- 3 396 721
- US-A- 3 861 383
- US-A- 4 265 228
- US-A- 5 119 832
- US-A- 5 131 409
- US-A- 5 133 352
- PROCEEDINGS OF THE IEEE 1990 ULTRASONICS SYMPOSIUM, Bd.3, 7. Dezember 1990, HONOLULU Seiten 1653 - 1656 J.CHAPELON 'thresholds for tissue ablation by focused ultrasound'
- Katalogblätter zu Piezolith 2500/R.Wolf Gmbh, 1992
- Gebrauchsanweisung zu Piezolith 2500, 1990

## Beschreibung

Die Erfindung betrifft ein Gerät zur Behandlung von Schmerzzuständen und/oder zur Beeinflussung des vegetativen Nervensystems, aufweisend eine Quelle impulsartiger Wellen, mittels derer wenigstens eine impulsartige Welle auf einen neural sensiblen Bereich geleitet wird, der bezüglich des Schmerzes bzw. bezüglich des zu beeinflussenden Bereiches des vegetativen Nervensystems an der Reizleitung beteiligt ist, wobei Betätigungsmittel vorgesehen sind, mittels derer die Quelle mit einer solchen Folgefrequenz der impulsartigen Wellen betätigbar ist, daß der zeitliche Abstand zwischen zwei aufeinanderfolgenden impulsartigen Wellen mehrere Minuten nicht unterschreitet.

Derartige Geräte sind in Anbetracht der hohen Zahl von Patienten, die unter Schmerzzuständen unterschiedlichster Art leiden, von großer Bedeutung, da die Einnahme von Analgetika Suchtgefahren in sich birgt. Außerdem stellen Analgetika insbesondere bei lange andauernder Einnahme eine erhebliche Belastung für den Stoffwechsel des Patienten dar. Es können sogar Schädigungen der Stoffwechselorgane, z.B. der Nieren, auftreten. Es kommt hinzu, daß in vielen Fällen die Analgetika mit zunehmender Behandlung höher dosiert werden müssen, um den Behandlungserfolg zu gewährleisten. Außerdem gibt es eine hohe Zahl von Patienten mit Analgetika-Unverträglichkeit bzw. -Allergie. Ebenfalls von besonderer Bedeutung sind Geräte der eingangs genannten Art bei der Behandlung von Drogensüchtigen während des Entzugs im Hinblick auf die dabei auftretenden Schmerzen und sonstigen Entzugserscheinungen. Schließlich sind Geräte der eingangs genannten Art auch im Hinblick auf die hohe Zahl von Patienten mit vegetativen Störungen, z.B. Herzrhythmusstörungen, Verdauungsstörungen, Schweißausbrüchen, Verspannungen u.dgl. von Bedeutung.

Geräte der eingangs genannten Art erlauben nämlich Schmerztherapie und Behandlung von vegetativen Störungen gänzlich ohne, oder zumindest mit verminderter Medikation.

Ein als Atlastherapie bekannt gewordenes Verfahren geht auf den französischen Arzt Arlen zurück (siehe "Manuelle Medizin", 27. Jhrg., Heft 4, Auf. 1989, Seite 82, Springer Verlag). Bei der Atlastherapie wird durch manuelle Impulse (Manipulation), insbesondere Schlag mit Finger, auf einen der Querfortsätze des ersten Halswirbels die Therapie von Schmerzen im Wirbelsäulenbereich ermöglicht. Außerdem werden Verspannungen im Wirbelsäulenbereich nachhaltig positiv beeinflußt, also vermindert oder gänzlich behoben. Auch erste Folgeerscheinungen der multiplen Sklerose können durch die Atlastherapie gebessert werden, so daß z.B. eine akute Rückbildung von Paresen (Lähmungen) möglich ist. Außerdem wurde festgestellt, daß durch diese Therapie auch vegetative Reize ausgelöst werden, z.B. die Durchblutung deutlich verbessert wird.

Es konnte biochemisch nachgewiesen werden, daß es im Zusammenhang mit der Atlastherapie zu einer signifikanten Änderung der Neurotransmitter (z.B. Dopamin) als auch der biogenen Amine kommt, was durch biochemische Bestimmungen im Blut nachgewiesen wurde. Dabei beschränkt sich die Änderung der Neurotransmitter und biogenen Amine nicht nur auf den Therapiebereich, sondern ist im gesamten Organismus feststellbar.

Nachteilig an der Atlastherapie ist, daß sie manuell ausgeführt werden muß und somit subjektiven Einflüssen unterliegt, also nicht reproduzierbar ist.

Ein Gerät der eingangs genannten Art, das zur Beeinflussung des vegetativen Nervensystems dient, ist aus der US-PS 3 396 721 bekannt.

Außerdem ist aus es der US-PS 3 499 437 der bekannt, eine Folge von impulsartigen Ultraschallwellen sowohl zur Schmerztherapie als auch zur Beeinflussung des vegetativen Nervensystems in periphere Bereiche des Nervensystems zu einzuleiten, die bezüglich des jeweils relevanten Geschehens an der Reizleitung beteiligt sind. Es soll auf diese Weise eine Mikromassage bewirkt werden. Nachteilig an diesem Verfahren ist, daß es infolge seiner peripheren Anwendung (ähnlich wie Reizstrom), wenn überhaupt, dann nur nach häufiger, jeweils länger andauernder Anwendung zu einem Erfolg führt.

Die US-A-5 133 352 betrifft ein Gerät zur Behandlung von Herpes. Dabei wird auf elektrochemische Vorgänge im Körper des Patienten eingewirkt, indem Gleichstrom in den Körper des Patienten eingeleitet wird. Die Einleitung von Gleichstrom erfolgt dabei jeweils für die Dauer von zwei bis zwanzig Sekunden in zeitlichen Abständen von einer Stunde.

Der Erfindung liegt daher die Aufgabe zugrunde, ein Gerät der eingangs genannten Art anzugeben, bei dem Energiegehalt und zeitlicher Verlauf der impulsartigen Welle gut reproduzierbar sind.

In Katalogblättern zu dem Gerät Piezolith 2500 der Firma Richard Wolf sowie einer Gebrauchsanweisung für dieses Gerät ist ein Lithotripter beschrieben, der impulsartige Wellen einer 100 ms nicht übersteigenden Dauer erzeugt und Betätigungsmittel in Form eines Handtasters aufweist, mittels dessen einzelne impulsartige Wellen auslösbar sind.

Nach der Erfindung wird die Aufgabe dadurch gelöst, daß die Dauer der impulsartigen Wellen 100 ms nicht übersteigt und daß die Folgefrequenz der impulsartigen Wellen mittels der Betätigungsmittel einstellbar ist. Die Erfindung beruht auf der Erkenntnis, daß Wirksamkeit der Atlastherapie darauf beruht, daß durch den Fingerschlag ein neural sensibler Bereich, nämlich das Ganglion cervicale superior, umstimuliert wird mit der Folge, der Umstellung der von diesen Ganglion abhängigen Nervenbahnen, was zur Beseitigung oder einem Nachlassen der Schmerzempfindungen und zu einem Abbau der Verspannungen führt. Im Vergleich zur manuellen Therapie sind im Falle der Erfindung der Energiegehalt und der zeitliche Verlauf der impulsartigen Welle sehr gut reproduzierbar. Außerdem wird der wesentliche Vorteil erzielt, daß mit einem einzigen Impuls wesentlich höhere Energien als im Falle der manuellen Therapie dem Körper des Patienten zugeführt werden können, so daß auch tiefliegende neural sensible Bereiche erreichbar sind. Unter einer impulsartigen Welle soll im vorliegenden Falle ein Impuls verstanden werden, bei dem es sich im wesentlichen um eine einmalige, vorzugsweise von einem Ruhezustand aus erfolgende impulsartige Amplitudenänderung handelt. In aller Regel ist übrigens der neural sensible Bereich nicht mit dem schmerzenden bzw. zu beeinflussenden Bereich identisch, d.h. der schmerzende oder zu beeinflussende Bereich wird nicht direkt mit den akustischen Druckimpulsen beaufschlagt. Dies kann einen wesentlichen Vorteil darstellen, da ein etwaiges Krankheitsgeschehen in dem schmerzenden bzw. zu beeinflussenden Bereich durch die Druckimpulsanwendung nicht unmittelbar beeinflußt werden kann. Außerdem ist wesentlich, daß nicht eine Folge von impulsartigen Wellen eingeleitet wird, sondern pro Behandlung nur eine einzige impulsartige Welle. Eine Folge von impulsartigen Wellen wäre wenig hilfreich und unter Umständen sogar schädlich, weil durch eine Folge von impulsartigen Wellen nicht sichergestellt werden kann, daß die erforderliche "Umstimulation" erfolgt. Allenfalls ist es denkbar, nach mehreren Minuten eine zweite impulsartige Welle einzuleiten, wenn die erste zu keinem spürbaren Erfolg geführt hat.

Mittels der Erfindung können Schmerzzustände und Verspannungen im Bereich der Wirbelsäule gut behandelt werden, indem ein dicht bei einem Querfortsatz des ersten Halswirbels liegender neural sensibler Bereich, z.B. das Ganglion cervicale superior , mit der impulsartigen Welle beaufschlagt wird. Als neural sensibler Bereich, der als Ansatzpunkt besonders geeignet ist, kommt ein Ganglion der aus Ganglion coeliacum , Ganglion cervicale superior, Ganglion sacrale und Ganglion pelvinum enthaltenden Gruppe in Frage.

Es wird davon ausgegangen, daß durch die Beaufschlagung mit eine einer impulsartigen Welle sozusagen eine "Umstimulation" des jeweiligen Ganglions erfolgt, wodurch Schmerzfreiheit bzw. eine Beeinflussung der an das jeweilige Ganglion angebundenen Bereich des vegetativen Nervensystems erreicht wird.

Im Gegensatz zu anderen "Manipulationen" von peripheren Nervenzellen, sei es durch Reizstrom, Massage oder auch durch Schallimpulse, greift die vorliegende Erfindung an den zentral regulierenden Steuermechanismen von Nervenschaltstellen, nämlich Nervenzentren und insbesondere Ganglien und nicht an peripheren, im schmerzenden bzw. zu beeinflussenden Bereich liegenden Nervenreizpunkten an. Mit der vorliegenden Erfindung kann z.B. durch die Beeinflussung des Ganglion coeliacum auch bei gegen medikamentöse Therapien praktisch resistenten Schmerzen Schmerzstillung erreicht werden. So können z.B. durch eine Pankreatitis (Morbus Wipple) oder andere Erkrankungen im Oberbauch ausgelöste Schmerzen behoben werden. Insbesondere derartige Schmerzen sind fast immer therapieresistent und benötigen hohe Dosen an Morphinpräparaten. Auch Schmerzen im Lendenwirbelbereich (Lendenwirbelsäulen-Syndrom) und Schmerzen im Sakralbereich sind durch Beeinflussung des Ganglion pelvinum gut therapierbar.

Wesentlich ist, daß die auf die Ganglien geleiteten Impulse nur dann ihren Effekt erzielen können, wenn sie ausreichend kurz sind, d.h. 100 ms (Millisekunden) nicht übersteigen. Vorzugsweise sollte die Impulsdauer 70 ms nicht übersteigen.

Ergänzend ist zu sagen, daß sich die Erfindung beispielsweise für die Behandlung folgender Leiden bzw. im Zusammenhang mit diesen Leiden auftretender Schmerzen eignet:
1. Schmerzzustände bei rheumatoiden Erkrankungen und Verspannungen,
2. Schmerzzustände bei Durchblutungsstörungen (z.B. der Beine als Folge von Stoffwechselerkrankungen oder im Falle von vasospastischen Erkrankungen usw.),
3. Schmerzzustände bei internistischen Erkrankungen (z.B. Morbus Wipple, Angina Pectoris, Magenschmerzen, funktionelle Oberbauchbeschwerden usw.),
4. Schmerzzustände bei neurologischen und psychiatrischen Erkrankungen (Neuralgien, Multiple Sklerose, neurovegetatives Syndrom, Verspannungen usw.), und
5. Schmerzzustände bei Tumorerkrankungen und Begleittherapie bei der Tumortherapie.

Während eine einmalige Beaufschlagung des jeweiligen Ganglion mit einem akustischen Druckimpuls bzw. einer impulsartigen elektromagnetischen Welle in der Regel nur einen kurzfristigen Erfolg bringt, ist bei einer mehrmaligen, z.B. dreimaligen, Wiederholung der Behandlung, beispielsweise im Wochenabstand, ein längerfristiger Erfolg (Monate) erreichbar, in denen der Patient auch ohne Analgetika leben kann. Die Wiederholung der Behandlung ist nicht mit der Verabreichung einer Folge impulsartiger Wellen zu verwechseln.

Unter Umständen kann es zweckmäßig sein, gleichzeitig mit der Behandlung durch die impulsartigen Wellen eine medikamentöse Therapie durchzuführen, beispielsweise durch Gabe von Lokalanästhetika ähnlich wie im Falle der klassischen Neuraltherapie.

Praktisch wichtig für die Anwendung der Erfindung ist, daß infolge der segmentalen Versorgung der inneren Organe bei Erkrankung eines inneren Organes eine Überempfindlichkeit und Schmerzhaftigkeit (Hyperästhesie und Hyperalgesie) der zugehörigen Hautbezirke auftritt. Der Grund hierfür ist, daß die afferenten hinteren Wurzeln der Spinalnerven nicht nur afferente, vegetative Fasern aus den jeweils zugehörigen Organbezirken, sondern auch von Hautsegmenten (Dermatomen) führen. Anhand der im Zusammenhang mit einer zu behandelnden Erkrankung auftretenden überempfindlichen und schmerzhaften Hautbezirke, die auch als Head'sche Zonen bezeichnet werden, kann also das für die jeweilige Erkrankung maßgebliche Ganglion ermittelt und mit der impulsartigen Welle beaufschlagt werden. Weiter besteht auch die Möglichkeit, eine oder mehrere Head'sche Zonen selbst als neural sensible(n) Bereich(e) mit der impulsartigen Welle zu beaufschlagen, um das zugehörige Organ bzw. die zugehörigen Organe zu therapeutischen und/oder diagnostischen Zwecken zu beeinflussen.

Um zu erreichen, daß sich die Wirkung der impulsartigen Welle nur auf den jeweiligen neural sensiblen Bereich beschränkt, und um zu gewährleisten, daß auch dann, wenn tiefer liegende neural sensible Bereiche erreicht werden sollen, die dem Patienten zugeführte Dosis an Energie nicht unnötig hoch ist, ist gemäß einer bevorzugten Variante der Erfindung vorgesehen, daß die impulsartige Welle fokussiert wird.

Wie auch im Falle der manuellen Therapie tritt nach der Behandlung eines Patienten mit dem erfindungsgemäßen Gerät nicht nur lokal sondern im gesamten Organismus eine Änderung der Neurotransmitter sowie der biogenen Amine auf. Die entsprechenden Untersuchungen können nach der bei W. Kloepfer & Weth, G. et al., "Atlastherapie and Neurotransmitters in Patients Affected by Multiple Sclerosis", Manuelle Medizin, 27. Jahrgang, Heft 4, Seite 82, August 1989, Springer Verlag, beschriebenen Methode durchgeführt werden.

Als besonders erfolgreich hat es sich erwiesen, als impulsartige Welle eine mechanische Welle auf den neural sensiblen Bereich zu leiten, die vorzugsweise mittels einer akustischen Druckimpulsquelle erzeugt wird.

Eine Variante der Erfindung sieht in diesem Zusammenhang vor, daß die Körperoberfläche des Patienten mittels der Druckimpulsquelle zur Erzeugung der mechanischen Welle mit einem Festkörper stoßartig beaufschlagt wird. Ein entsprechendes Gerät kann handlich und einfach aufgebaut sein. Dabei kann vorgesehen sein, daß die Druckimpulsquelle in aus der US-A-3 396 721 und aus der US-A-4 265 228 an sich bekannter Weise als Festkörper einen beispielsweise elektromagnetisch stoßartig antreibbaren Stößel enthält, durch dessen Auftreffen auf die Körperoberfläche die mechanische Welle erzeugt wird. Die Beaufschlagung der Körperoberfläche mit dem Stößel kann direkt oder unter Zwischenfügung eines akustischen Koppelmediums erfolgen. Es kann aber auch vorgesehen sein, daß die Körperoberfläche des Patienten zur Erzeugung der mechanischen Welle mittels eines von der Druckimpulsquelle abgegebenen Geschosses beaufschlagt wird. Als Geschosse eignen sich beispielsweise Hohlkugeln aus Kunststoff, Schaumstoffkugeln, Korkkugeln etc.. Es versteht sich, daß die Energie des Geschosses so gering ist, daß dem Patienten keinerlei Verletzungen zugefügt werden.

Vorzugsweise beträgt die kinetische Energie des Festkörpers wenigstens 5 Millijoule . Die Geschwindigkeit des Festkörpers sollte wenigstens 3 m/s betragen, da dann mechanische Wellen in den Körper des Patienten eingeleitet werden, deren Dauer höchstens 100 ms beträgt. Die Masse des Festkörpers sollte wenigstens 1 g betragen, da sich dann die zur Erzeugung der genannten kinetischen Energie erforderliche Geschwindigkeit des Festkörpers in Grenzen hält.

Gemäß einer weiteren Ausführungsform der Erfindung wird die Körperoberfläche des Patienten mittels der Druckimpulsquelle zur Erzeugung der mechanischen Welle in aus der US-A-3 861 383 an sich bekannter Weise mit einem Fluid impulsartig beaufschlagt. Die impulsartige Beaufschlagung mit dem Fluid entspricht in ihrem Effekt der Beaufschlagung der Körperoberfläche mit einem Geschoß. Zweckmäßigerweise enthält dann die Druckimpulsquelle ein mit der umgebenden Atmosphäre in über eine Öffnung in Verbindung stehendes, schlagartig verkleinerbares, mit einem Fluid gefülltes Volumen, wobei das die Körperoberfläche des Patienten beaufschlagende Fluid durch die Öffnung abgegeben wird, wenn das Volumen schlagartig verkleinert wird. Als Fluid kann sowohl ein Gas als auch eine Flüssigkeit Verwendung finden.

Nach einer besonders bevorzugten Ausführungsform wird die mechanische Welle mittels einer elektromagnetischen Druckimpulsquelle erzeugt, und zwar vorzugsweise in Form einer akustischen Stoßwelle. Es handelt sich hierbei um einen positiven akustischen Druckimpuls mit extrem steiler Anstiegsflanke. Die Verwendung einer aus der EP-A-0 301 360 an sich bekannten elektromagnetischen Druckimpulsquelle ist deshalb besonders vorteilhaft, weil derartige Druckimpulsquellen eine sehr feinfühlige und gut reproduzierbare Einstellung der akustischen Eigenschaften der erzeugten Druckimpulse ermöglichen. Es können jedoch, und dies gilt auch für die Erzeugung von Stoßwellen, auch andere Druckimpulsquellen Verwendung finden. Es seien hier beispielhaft piezoelektrische, elektrohydraulische und magnetostriktive Impulsquellen erwähnt.

Für die mechanischen Welle bzw. den Druckimpuls gilt, daß deren Amplitude dem Betrag nach im Vergleich zur Amplitude etwaiger Ausschwingvorgänge groß (> Faktor 3) ist. Obwohl bevorzugt positive mechanische Wellen bzw. Druckimpulse (Überdruck) vorgesehen sind, ist es grundsätzlich auch möglich, negative Druckimpulse (Unterdruck) anzuwenden, sofern sichergestellt ist, daß Schädigungen des jeweiligen neural sensiblen Bereiches durch Kavitation ausgeschlossen sind. Schädigungen des jeweiligen neural sensiblen Bereiches durch thermische Einflüsse sind praktisch ausgeschlossen, da mechanische Wellen bzw. akustische Druckimpulse der genannten Art von keinen nennenswerten thermischen Erscheinungen begleitet sind und lediglich singulär zur Anwendung kommen.

Gemäß einer weiteren Variante der Erfindung wird eine elektromagnetische Welle auf den neural sensiblen Bereich geleitet. Auch hier sind insbesondere im Falle der Fokussierung der elektromagnetischen Welle tiefer liegende neural sensible Bereiche behandelbar, wobei auch die impulsartige elektromagnetische Welle hinsichtlich ihres Energiegehaltes sowie des zeitlichen Verlaufes ihrer Amplitude gut reproduzierbar ist. Auch eine Gefahr von insbesondere thermischen Schädigungen des jeweiligen neuralsensiblen Bereiches ist nicht gegeben, da die elektromagnetischen Wellen wie auch die mechanischen Wellen singulär angewendet werden.

Es besteht auch die Möglichkeit, gemäß einer Ausführungsform der Erfindung als impulsartige Welle einen elektrischen Impuls, wie er beispielsweise bei einer elektrischen Entladung, z.B. Kondensatorentladung, auftritt, auf den neural sensiblen Bereich zu leiten. Dies ist besonders dann von Vorteil, wenn die impulsartige Welle mittels eines in den Patienten implantierbaren Gerätes erzeugt werden soll. Ein derartiges implantierbares Gerät kann ähnlich wie ein implantierbarer Defibrillator oder Herzschrittmacher aufgebaut sein, wobei der elektrische Impuls dem jeweiligen neural sensiblen Bereich über eine katheterartige Elektrode zugeführt werden kann. Im Gegensatz zu einem Defibrillator oder Herzschrittmacher oder einer aus der US-A-5 119 832 bekannten Einrichtung zur Schmerzbehandlung wird aber eine Folge elektrischer Impulse oder ein elektrischer Impuls nicht beim Auftreten bzw. Ausbleiben eines physiologischen Ereignisses abgeben. Vielmehr werden in der zuvor beschriebenen Weise nur einzelne Impulse appliziert, die vom behandelnden Arzt oder auch vom Patienten selbst ausgelöst werden können, beispielsweise mittels einer drahtlosen Fernbedienung.

Auch im Falle der Anwendung elektromagnetischer Wellen oder elektrischer Impulse beschränkt sich die Anwendung in der Regel auf einen Bereich, der von dem schmerzenden bzw. zu beeinflussenden Bereich verschieden ist.

Ausführungsbeispiele der Erfindung sind in den beigefügten Zeichnungen dargestellt. Es zeigen:
- Fig. 1: die Behandlung eines Patienten mittels einer akustischen Stoßwellenquelle, und
- Fig. 2: die Druckimpulsquelle gemäß Fig. 1 im Längsschnitt.

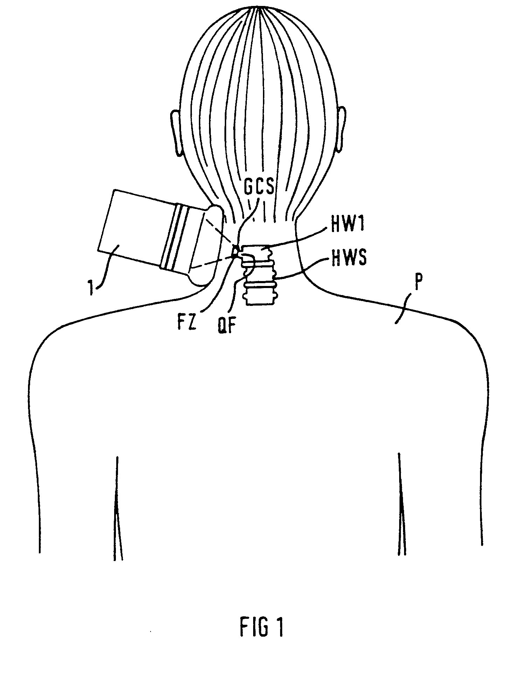

In Fig. 1 ist ein mit P bezeichneter Patient dargestellt, der im Bereich seiner Wirbelsäule unter Schmerzen und Verspannungen leidet.

In Fig. 1 ist die Halswirbelsäule HWS des Patienten P schematisch angedeutet. Dabei ist aus der Fig. 1 ersichtlich, daß der erste Halswirbel HW1 eine Verschiebung, und zwar nach links (aus Sicht des Betrachters), aufweist.

Zur Behandlung seiner Beschwerden mit dem erfindungsgemäßen Gerät, von letzterem in Fig. 1 nur die insgesamt mit 1 bezeichnete Druckimpulsquelle dargestellt ist, wird mittels der Druckimpulsquelle 1 in den Patienten P eine impulsartige mechanische Welle, nämlich ein akustischer Druckimpuls in Form einer Stoßwelle, eingeleitet. Dabei wird die akustische Druckimpulsquelle 1 mittels einer flexiblen Ankoppelmembran von links an den Hals des Patienten akustisch angekoppelt, und zwar derart, daß die Fokuszone FZ der Stoßwelle, die Randstrahlen der Stoßwelle sind in Fig. 1 strichliert angedeutet, im Bereich des linken Querfortsatzes QF des verschobenen ersten Halswirbels HW1 liegt. Wird nun eine Stoßwelle ausgelöst, trifft diese das im Bereich des Querfortsatzes QF des betroffenen Halswirbels HW1 liegenden Ganglion cervicale superior GCS, das in Fig. 1 schematisch angedeutet ist.

Durch das Auftreffen der Stoßwelle wird das Ganglion cervicale superior umstimuliert, mit der Folge der Umstellung der vom Ganglion cervicale superior abhängigen Nervenbahnen. Dies führt dazu, daß die Schmerzempfindung zumindest nachläßt und sich die Verspannungen abbauen. Der Behandlungserfolg tritt praktisch unmittelbar nach der Stoßwellenanwendung ein.

Um einen länger andauernden Behandlungserfolg erreichen zu können, reicht eine einmalige Behandlung nicht aus, vielmehr ist eine mehrfache, beispielsweise dreifache, Behandlung möglich, wobei die einzelnen Behandlungen etwa im Wochenabstand liegen sollten.

In der Regel wird es genügen, pro Behandlung eine einzige Stoßwelle zu applizieren. Grundsätzlich ist es jedoch auch möglich, Folgen von Stoßwellen zu applizieren.

Geeignete Druckimpulsquellen sind z.B. in der US-PS 4 697 588, der US-PS 4 764 505 und der EP-A-0 301 360 beschrieben.

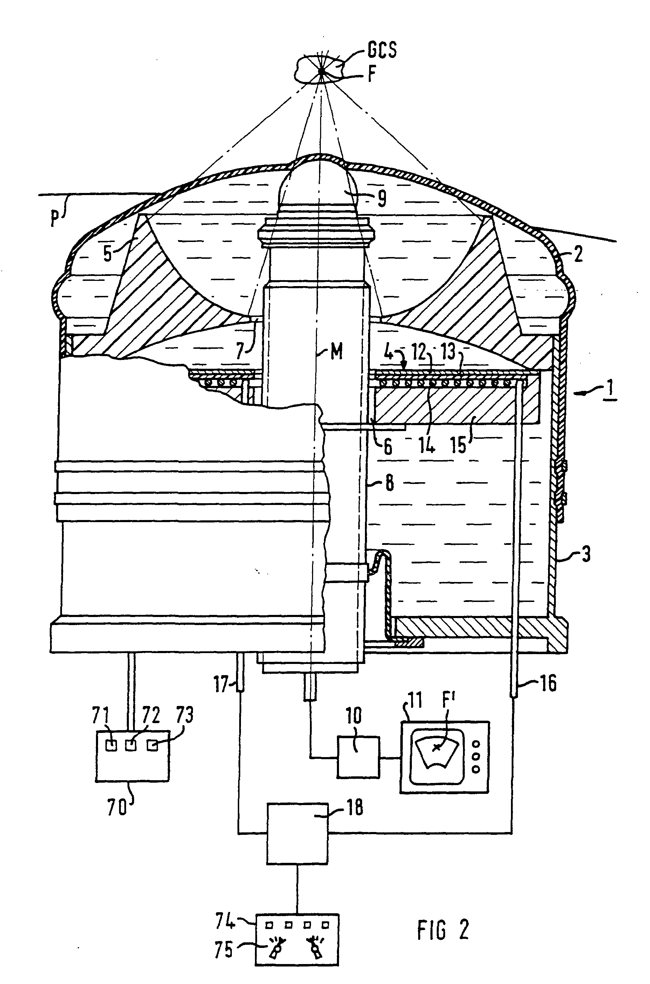

Der Aufbau der Druckimpulsquelle 1 wird im folgenden beispielhaft anhand der Fig. 2 näher erläutert.

Die Druckimpulsquelle 1 gemäß Fig. 2 weist ein etwa topfförmiges Gehäuse 3 auf, im Bereich von dessen einem Ende ein insgesamt mit 4 bezeichneter Stoßwellengenerator vorgesehen ist. An seinem offenen Ende ist das Gehäuse 3 der übrigens im wesentlichen rotationssymmetrisch zur Mittelachse M ausgebildeten Druckimpulsquelle 1 mittels der flexiblen Koppelmembran 2 verschlossen. Mittels dieser wird die Druckimpulsquelle 1 zur akustischen Koppelung in der in Fig. 1 gezeigten Weise an den Körper des Patienten P angepreßt. Das Gehäuse 3 ist mit Wasser gefüllt, das als akustisches Ausbreitungsmedium für die von dem Stoßwellengenerator 4 ausgehenden Stoßwellen vorgesehen ist.

Bei dem Stoßwellengenerator 4 handelt es sich um einen elektromagnetischen Stoßwellengenerator. Dem Stoßwellengenerator 4 ist eine akustische Sammellinse 5 vorgelagert, die zur Fokussierung der von dem Stoßwellengenerator ausgehenden ebenen Stoßwellen dient, die dann wie in den Fig. 1 und 2 strichliert angedeutet, in der auf der Mittelachse M liegenden Fokuszone F zusammenlaufen. Der Stoßwellengenerator 4 und die Sammellinse 5 sind mit miteinander fluchtenden zentralen Öffnungen 6, 7 versehen, durch die sich ein Rohr 8 erstreckt, in dem ein Ultraschall-Applikator 9 angeordnet ist, der es gestattet, mit einer schematisch angedeuteten elektronischen Einrichtung 10 Ultraschall-B-Bilder einer die Mittelachse M und die Fokuszone F der Stoßwellen enthaltenden Schicht des Körpers des Patienten zu erzeugen

Mittels der durch den Ultraschall-Applikator 9 und die elektronische Einrichtung 10 gebildeten Ultraschall-Ortungseinrichtung und/oder einer nicht dargestellten, an sich bekannten Röntgen-Ortungseinrichtung ist es in an sich bekannter Weise möglich, die Druckimpulsquelle 1 relativ zum Körper des Patienten so auszurichten, daß sich ein mit Stoßwellen zu beaufschlagender Bereich, beispielsweise das der Querfortsatz QF bzw. das Ganglion cervicale superior GCS, so wie dies in Fig. 1 dargestellt ist, in der Fokuszone F der Stoßwellen befindet. Dies geschieht mit Hilfe einer die Lage der Fokuszone F kennzeichnenden Marke F', die in das auf einem Monitor 11 angezeigte Ultraschall-B-Bild in an sich bekannter Weise eingeblendet ist.

Um die Druckimpulsquelle 1 in der beschriebenen Weise relativ zu dem Patienten ausrichten zu können, ist sie mittels einer nicht näher dargestellten, an sich bekannten Verstelleinrichtung 70 mit Bedienelementen 71, 72, 73 räumlich verstellbar. Sie kann somit relativ zu dem stationären Körper des Patienten P so ausgerichtet werden, daß der Fokus F der Stößwellen so wie in Fig. 1 dargestellt innerhalb des neural sensiblen Bereiches GCS liegt. Dies geschieht mit Hilfe der die Lage des Fokus F kennzeichnenden Marke F'. Die "Randstrahlen" der Stoßwellen sind in den Figuren strichliert angedeutet.

Andere Verstellmöglichkeiten, z.B. Verstellung nur des Patienten P oder Verstellung sowohl des Patienten P als auch der Druckimpulsquelle 1, sind möglich, um den Patienten P bzw. den neural sensiblen Bereich GCS einerseits und die Druckimpulsquelle 1 bzw. den Fokus F andererseits in der erforderlichen Weise relativ zueinander verstellen zu können.

Der Ultraschall-Applikator 9 ist übrigens in Richtung der Mittelachse M in dem Rohr 8 längsverschieblich sowie um die Mittelachse M schwenkbar angeordnet, um in Abhängigkeit von den besonderen Gegebenheiten des jeweiligen Behandlungsfalles ein möglichst wenig gestörtes, informatives Ultraschall-B-Bild erzeugen zu können und um das kuppelförmige Schallaustrittsfenster des Ultraschall-Applikators 9 in der für eine gute Bildqualität erforderlichen Weise mit der an das akustische Ausbreitungsmedium angrenzenden Seite der Koppelmembran 2 in Eingriff bringen zu können, während deren andere Seite an der Körperoberfläche des Patienten anliegt. Dabei wird die Lage der Marke F' in an sich bekannter Weise der jeweiligen Stellung des Ultraschall-Applikators 9 angepaßt. Die zu der beschriebenen Verstellung des Ultraschall-Applikators 9 erforderlichen Verstelleinheiten können vom Fachmann ohne weiteres realisiert werden und sind daher nicht dargestellt.

Der Stoßwellengenerator 4 weist eine elektrisch leitfähiges Material, beispielsweise Kupfer oder Aluminium, enthaltende ebene Membran 12 auf, deren eine Seite an das in dem Gehäuse 3 befindliche Wasser angrenzt. Die andere Seite der Membran 12 liegt unter Zwischenfügung einer Isolierfolie 13 an einer spiralförmig gewickelten Flächenspule 14 an, die beispielsweise durch Kleben auf einem Spulenträger 15 angebracht ist. Die Flächenspule 14 steht über Anschlüsse 16 und 17 mit einem Hochspannungsimpulsgenerator 18 in Verbindung, mittels dessen die Flachspule 14 mit Hochspannungsimpulsen hoher Stromstärke (kV- und kA-Bereich) beaufschlagbar ist. Der Hochspannungsimpulsgenerator 18 ist so ausgebildet, daß die Intensität der Stoßwellen und die Folgefrequenz der Stoßwellen einstellbar sind. Zu diesem Zweck ist eine Steuereinheit 74 mit einem Bedienfeld 75 vorgesehen, die an den Hochspannungsimpulsgenerator 18 angeschlossen ist und die Einstellung der genannten Parameter erlaubt.

Bei Beaufschlagung der Flächenspule 14 mit einem Hochspannungsimpuls werden in die Membran 12 Wirbelströme induziert, die dem durch die Flächenspule 14 fließenden Strom entgegengesetzt gerichtet sind. Dies hat zur Folge, daß das zu den Wirbelströmen gehörige Magnetfeld und das zu dem durch die Flächenspule 14 fließenden Strom gehörigen Magnetfeld entgegengesetzt gerichtet sind. Somit bewegt sich die Membran 12 schlagartig von der Flächenspule 14 weg. Hierdurch bildet sich in dem in dem Gehäuse 3 befindlichen Wasser eine Stoßwelle aus, die mittels der akustischen Sammellinse 5 fokussiert wird. Die Impulsdauer der erzeugten Stoßwelle liegt im µs-Bereich, ist also deutlich geringer als 100 ms. Die Energie der erzeugten Stoßwelle kann in der Größenordnung von bis zu 120 Millijoule liegen, wobei die Energiedichte im Fokus bis zu ca. 0,6 Millijoule/mm² betragen kann. Der Spitzendruck im Fokus kann bis zu 700 bar betragen.

Obwohl es besonders vorteilhaft ist, eine elektromagnetische Druckimpulsquelle der beschriebenen Art zu verwenden, da diese gut regelbar sind, können auch andere Druckimpulsquellen, beispielsweise piezoelektrische (US-PS 4 526 168), magnetostriktive, elektrohydraulische (DE-OS 23 51 247) u.a. Stoßwellenquellen, im Rahmen der Erfindung verwendet werden. Auch muß es sich bei den im Rahmen der Erfindung verwendeten Druckimpulsquellen nicht notwendigerweise um Stoßwellenquellen handeln.

Auch akustische Druckimpulsquellen, die nicht als Stoßwellen einzuordnende akustische Druckimpulse erzeugen, können Verwendung finden. Als Beispiel sei nur auf solche Druckimpulsquellen verwiesen, die akustische Unterdruckimpulse erzeugen.

## Patentansprüche

1. Gerät zur Behandlung von Schmerzzuständen und/oder zur Beeinflussung des vegetativen Nervensystems, aufweisend eine Quelle (1) impulsartiger Wellen, mittels derer impulsartige Wellen auf einen neural sensiblen Bereich (GC, GCS, GP, GS) geleitet werden, der bezüglich des Schmerzes bzw. bezüglich des zu beeinflussenden Bereiches des vegetativen Nervensystems an der Reizleitung beteiligt ist, wobei Betätigungsmittel (74) vorgesehen sind, mittels derer die Quelle (1) mit einer solchen Folgefrequenz der impulsartigen Wellen betätigbar ist, dass der zeitliche Abstand zwischen zwei aufeinanderfolgenden impulsartigen Wellen mehrere Minuten nicht unterschreitet, **dadurch gekennzeichnet , daß** die Dauer der impulsartigen Wellen 100 ms nicht übersteigt und dass die Folgefrequenz der impulsartigen Wellen mittels der Betätigungsmittel (74) einstellbar ist.

2. Gerät nach Anspruch 1, welches implantierbar ist.

3. Gerät nach Anspruch 1 oder 2, welches Fokussierungsmittel für die impulsartige Welle aufweist.

4. Gerät nach einem der Ansprüche 1 bis 3, welches als imimpulsartige Welle eine mechanische Welle erzeugt.

5. Gerät nach Anspruch 4, welches als Quelle impulsartiger Wellen eine Druckimpulsquelle (1) enthält, die als mechanische Welle einen Druckimpuls erzeugt.

6. Gerät nach Anspruch 5, das als Druckimpulsquelle eine elektromagnetische Druckimpulsquelle (1) enthält.

7. Gerät nach Anspruch 6, dessen Druckimpulsquelle (1) als Druckimpuls eine akustische Stoßwelle erzeugt.

8. Gerät nach einem der Ansprüche 1 bis 3, welches als Quelle impulsartiger Wellen eine Quelle elektromagnetischer Wellen enthält, mittels derer als impulsartige Welle eine elektromagnetische Welle erzeugbar ist.

9. Gerät nach einem der Ansprüche 1 bis 3, welches als Quelle impulsartiger Wellen eine Quelle elektrischer Impulse enthält, mittels derer als impulsartige Welle ein elektrischer Impuls erzeugbar ist.

10. Gerät nach Anspruch 9, welches implantierbar ist und eine Elektrode aufweist, mittels derer der elektrische Impuls dem neural sensiblen Bereich zuführbar ist.

## Claims

1. Apparatus for treating attacks of pain and/or for influencing the autonomic nervous system, having a source (1) of pulse-like waves, by means of which source pulse-like waves are directed to a neurally sensitive region (GC, GCS, GP, GS) which participates in the conduction with regard to the pain or with regard to the region of the autonomic nervous system that is to be influenced, with actuating means (74) being provided by means of which the source (1) can be actuated at such a repetition rate of the pulse-like waves that the temporal interval between two successive pulse-like waves does not fall below several minutes, **characterized in that** the duration of the pulse-like waves does not exceed 100 ms and that the repetition rate of the pulse-like waves can be set using the actuating means (74).

2. Apparatus according to claim 1, which is implantable.

3. Apparatus according to claim 1 or 2, which has focussing means for the pulse-like wave.

4. Apparatus according to one of claims 1 to 3, which generates a mechanical wave as a pulse-like wave.

5. Apparatus according to claim 4, which contains as a source of pulse-like waves a pressure-pulse source (1) which generates a pressure pulse as a mechanical wave.

6. Apparatus according to claim 5, which contains an electromagnetic pressure-pulse source (1) as the pressure-pulse source.

7. Apparatus according to claim 6, the pressure-pulse source (1) of which generates an acoustic shock wave as the pressure pulse.

8. Apparatus according to one of claims 1 to 3, which contains as a source of pulse-like waves a source of electromagnetic waves, by means of which an electromagnetic wave can be generated as the pulse-like wave.

9. Apparatus according to one of claims 1 to 3, which contains as a source of pulse-like waves a source of electric pulses, by means of which an electric pulse can be generated as the pulse-like wave.

10. Apparatus according to claim 9, which is implantable and has an electrode by means of which the electric pulse can be fed to the neurally sensitive region.

## Revendications

1. Appareil pour traiter des états douloureux et/ou pour agir sur le système nerveux végétatif, comportant une source (1) d'ondes impulsionnelles au moyen de laquelle on envoie des ondes impulsionnelles sur une zone neurosensible (GC, GCS, GP, GS) qui participe à la conduction du stimulus en ce qui concerne la douleur ou la région du système nerveux végétatif sur laquelle il faut agir, des moyens de commande étant prévus à l'aide desquels on peut commander la source (1) avec une fréquence de répétition des ondes impulsionnelles telle que l'écart dans le temps entre deux ondes impulsionnelles successives n'est pas inférieur à quelques minutes, **caractérisé en ce que** la durée des ondes impulsionnelles ne dépasse pas 100 m s et **en ce que** la fréquence de répétition des ondes impulsionnelles peut être réglée à l'aide des moyens de commande.

2. Appareil selon la revendication 1, lequel appareil est implantable.

3. Appareil selon la revendication 1 ou 2, lequel appareil comporte des moyens de focalisation pour l'onde impulsionnelle.

4. Appareil selon l'une des revendications 1 à 3, lequel appareil produit comme onde impulsionnelle une onde mécanique.

5. Appareil selon la revendication 4, lequel appareil contient comme source d'ondes impulsionnelles une source d'impulsions de pression (1) qui produit comme onde mécanique une impulsion de pression.

6. Appareil selon la revendication 5, lequel appareil contient comme source d'impulsions de pression une source d'impulsions de pression électromagnétique (1).

7. Appareil selon la revendication 6, dont la source d'impulsions de pression (1) produit comme impulsion de pression une onde de choc acoustique.

8. Appareil selon l'une des revendications 1 à 3, lequel appareil contient comme source d'ondes impulsionnelles une source d'ondes électromagnétiques au moyen de laquelle on peut produire comme onde impulsionnelle une onde électromagnétique.

9. Appareil selon l'une des revendications 1 à 3, lequel appareil contient comme source d'ondes impulsionnelles une source d'impulsions électriques au moyen de laquelle on peut produire comme onde impulsionnelle une impulsion électrique.

10. Appareil selon la revendication 9, lequel appareil est implantable et comporte une électrode au moyen de laquelle l'impulsion électrique peut être envoyée à la zone neurosensible.
